# EUROPÄISCHE PATENTSCHRIFT

(11) **EP 0 644 186 B1**
(45) Veröffentlichungstag und Bekanntmachung des Hinweises auf die Patenterteilung: **02.01.1997**
(21) Anmeldenummer: 94104862.1
(22) Anmeldetag: 28.03.1994
(51) Int. Cl.: C07C 403/20

(54) **Verfahren zur Herstellung von 13-(Z)-Retinsäure**
Process for the preparation of 13-(Z)- retinoic acid.
Procédé pour la préparation d'acide 13-(Z)-retinoique.

(30) Priorität: 22.04.1993 DE 4313089
(43) Veröffentlichungstag der Anmeldung: 22.03.1995
(73) Patentinhaber: BASF Aktiengesellschaft, 67063 Ludwigshafen (DE)
(72) Erfinder: John, Michael, Dr., D-67061 Ludwigshafen (DE); Joachim, Paust, Dr., D-67141 Neuhofen (DE)

(56) Entgegenhaltungen:
- EP-A- 0 010 208
- EP-A- 0 111 325
- J. LABELLED COMPD. RADIOPHARM., Bd.17, Nr.3, 1980 Seiten 463 - 469 R.R. MUCCINO ET AL
- J. CHEM. SOC. (C), Nr.16, 1968 Seiten 1982 - 1983 C.F. GARBERS ET AL

## Beschreibung

Die Erfindung betrifft die Herstellung von 13-(Z)-Retinsäure (I) durch Umsetzen von 5-Hydroxy-4-methyl-2-(5H)-furanon (II; Butenolid) mit einem [3-Methyl-5-(2,6,6-trimethyl-1-cyclohexen-1-yl)-2,4-pentadienyl]-triarylphosphoniumsalz (C₁₅-Triarylphosphoniumsalz), und anschließende partielle Isomerisierung des erhaltenen Gemisches aus 13-(Z)- und 11,13-di-(Z)-Retinsäure (auch 13-cis- und 11,13-di-cis-Retinsäure oder -Vitamin-A-säure genannt).

13-(Z)-Retinsäure ist eine pharmakologisch bedeutungsvolle Substanz bei der Behandlung von Akne. Ihre Synthese ist wegen der Bildung von Doppelbindungsisomeren relativ schwierig.

So wird beispielsweise gemäß einer im J. Chem. Soc. (C) (1968), Seiten 1984-97, beschriebenen Synthese aus dem Butenolid II und einem C₁₅-Triarylphosphoniumsalz in Diethylether ein Gemisch aus 13-(Z)- und 11,13-di-(Z)-Retinsäure in einer Ausbeute von 66 bis 75 % der Theorie erhalten, in dem das 13-(Z)-Isomere nur in etwa 36 % enthalten ist. Eine selektive Isomerisierung der 11-(Z)-Doppelbindungen bei gleichzeitiger Anwesenheit einer 13-(Z)-Doppelbindung konnte nicht erreicht werden.

Weiterhin ist aus dem Journal of labelled compounds and radio-pharmaceuticals, Bd. XVII, No. 3 (1980) Seiten 463-69 die Herstellung von ¹⁴C-markierter 13-(Z)-Retinsäure durch Umsetzen von ¹⁴C-markiertem C₁₅-Triphenylphosphoniumsalz mit dem Butenolid II in Gegenwart von Kaliumhydrid in Tetrahydrofuran als Lösungsmittel bei Raumtemperatur bekannt. Nachteilig an diesem Verfahren ist, daß man mit dem leicht zerfallenden, an der Luft selbstentzündlichen KH arbeiten muß und daß die erzielten Ausbeuten nur etwa 62 % betragen.

Weiterhin ist aus EP-B1-0 111 325 ein Verfahren zur Herstellung von 13-(Z)-Retinsäure durch Kopplung eines C₁₅-Triarylphosphoniumsalzes mit dem Butenolid II und nachfolgende Isomerisierung in Gegenwart von Übergangsmetallkatalysatoren bekannt. Nachteilig an diesem an sich recht guten Verfahren ist, daß man die Wittig-Reaktionsstufe bei Temperaturen von vorzugsweise -30 bis -45°C durchführen muß, was sehr hohe Energiekosten bedingt. Außerdem ist eine aufwendige Extraktionsprozedur notwendig.

Die anschließende selektive Isomerisierung der 11,13-di-(Z)-Retinsäure erfolgt mit sehr guten Ausbeuten, jedoch führt die Anwendung der dort beschriebenen Isomerisierungskatalysatoren zu einer Verunreinigung des Wertproduktes mit Spuren von Übergangsmetallen, die zu Stabilitätsproblemen beim resultierenden Wertprodukt führen können. Eine Verunreinigung des Wertproduktes mit organischem Phosphor aus dem Zusatz von Triphenylphosphin zur Dekomplexierung des Katalysators macht zusätzliche Reinigungsschritte notwendig. Darüber hinaus dürfte die Behandlung des Wertproduktes mit Acetonitril als Lösungsmittel für den Isomerisierungskatalysator nicht unproblematisch sein.

Es war daher die Aufgabe der Erfindung das Verfahren zur Herstellung von 13-(Z)-Retinsäure durch Umsetzung eines C₁₅-Triaryphosphoniumsalzes und dem Butenolid II in Gegenwart eines Alkalimetallhydroxids in einem organischen Lösungsmittel und anschließende partielle Isomerisierung so zu verbessern, daß man die Wittig-Reaktion bei energetisch vorteilhafteren Temperaturen und ohne die übrigen Nachteile des Standes der Technik mit sehr guten Ausbeuten durchführen kann. Es war außerdem eine Aufgabe der Erfindung, ein vorteilhafteres Verfahren zur Isomerisierung eines Gemisches aus 13-(Z)- und 11,13-di-(Z)-Retinsäure unter Erhalt reiner 13-(Z)-Retinsäure zu entwickeln.

Es wurde nun überraschenderweise gefunden, daß man die Umsetzung eines C₁₅-Triarylphosphoniumhalogenids oder -hydrogensulfats mit dem Butenolid II auch bei Temperaturen von +10 bis -9°C mit Ausbeuten von bis zu 97,5 % d. Theorie durchführen kann, wenn man die Umsetzung in Dimethylformamid (DMF) als Lösungsmittel und mit Lithiumhydroxid als Alkalihydroxid durchführt und das Butenolid in ein Gemisch aus dem C₁₅-Triarylphosphoniumsalz und LiOH in DMF einträgt.

Dieses Ergebnis war sehr überraschend weil bei diesen Temperaturen mit anderen basischen Verbindungen, wie Kaliummethylat, Natriumcarbonat, Natriumhydroxid oder Kalium-tert.-butylat in anderen für diese Umsetzung bekannten Lösungsmitteln, wie niederen Alkanolen, Heptan/Wasser-Gemischen oder DMF nur Ausbeuten von etwa 21,3 bis 44,5 % d. Theorie erzielt werden konnten. Beispielsweise erhielt man bei einer Umsetzung in Gegenwart von NaOH in DMF unter sonst gleichen Bedingungen nur Ausbeuten von 35 % der Theorie an dem Retinsäure-Isomerengemisch.

Weiterhin wurde gefunden, daß man das bei der oben beschriebenen Wittig-Reaktion erhaltene Gemisch aus 13-(Z)- und 11,13-di-(Z)-Retinsäure sehr vorteilhaft selektiv zu 13-(Z)-Retinsäure isomerisieren kann, wenn man das Isomerengemisch in einem organischen Lösungsmittel, vorzugsweise in einem niederen Alkanol, in Gegenwart eines geeigneten Photosensibilisators mit Licht im Wellenlängenbereich zwischen etwa 200 und 600 nm bestrahlt.

Gegenstand der Erfindung ist daher ein Verfahren zur Herstellung von 13-(Z)-Retinsäure der allgemeinen Formel I 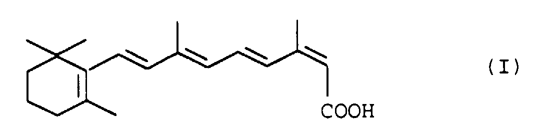 durch
a) Umsetzen von 5-Hydroxy-4-methyl-2-(5H)-furanon der Formel II 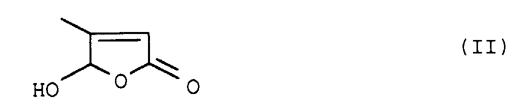 mit einem C₁₅-Triarylphosphoniumsalz der allgemeinen Formel III 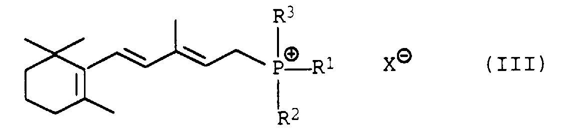 in der R¹, R² und R³ für einen Arylrest, vorzugsweise einen Phenylrest stehen und X^{⊖} für Halogen oder (HSO₄)⁻ steht, in Gegenwart eines Alkalimetallhydroxids in einem organischen Lösungsmittel und
b) anschließende partielle Isomerisierung des so erhaltenen Gemisches aus 13-(Z)- und 11,13-di-(Z)-Retinsäure,
   das dadurch gekennzeichnet ist, daß man im Reaktionsschritt a) das 5-Hydroxy-4-methyl-2-(5H)-furanon der Formel II mit einem Gemisch aus dem C₁₅-Triarylphosphoniumsalz der Formel III und 2 bis 6 Mol Lithiumhydroxid pro Mol III als Alkalimetallhydroxid in 2 bis 6 Litern Dimethylformamid als Lösungsmittel bei Temperaturen von +10 bis -9°C, vorzugsweise +5 bis -5°C, insbesondere -2 bis +2°C, umsetzt.

Besonders vorteilhaft gestaltet sich das erfindungsgemäße Verfahren wenn man im Verfahrensschritt b) zur Isomerisierung des Gemisches aus 13-(Z)- und 11,13-di-(Z)-Retinsäure in einem aliphatischen oder cycloaliphatischen Kohlenwasserstoff, einem halogenierten aliphatischen oder aromatischen Kohlenwasserstoff oder einem C₁- bis C₄-Alkanol als organischem Lösungsmittel, vorzugsweise einem C₁-C₄-Alkanol, insbesondere in Isopropanol, in Gegenwart eines Photosensibilisators, vorzugsweise in Gegenwart von Erytrosin B, mit Licht im Wellenlängenbereich zwischen 200 und 600 nm bestrahlt.

Gegenstand der Erfindung ist daher auch ein Verfahren zur Herstellung von 13-(Z)-Retinsäure der allgemeinen Formel I 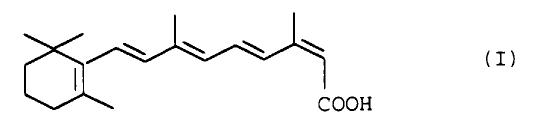 durch
a) Umsetzen von 5-Hydroxy-4-methyl-2-(5H)-furanon der Formel II 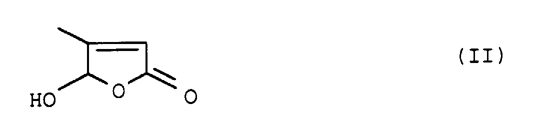 mit einem C₁₅-Triarylphosphoniumsalz der allgemeinen Formel III 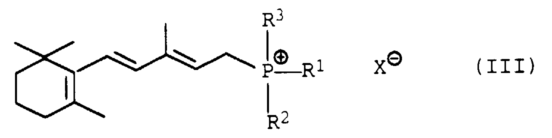 in der R¹, R² und R³ für einen Arylrest, vorzugsweise einen Phenylrest, stehen und X^{⊖} für Halogen oder (HSO₄)⁻ steht, in Gegenwart eines Alkalimetallhydroxids in einem organischen Lösungsmittel und
b) anschließende partielle Isomerisierung des so erhaltenen Gemisches aus 13-(Z)- und 11,13-di-(Z)-Retinsäure,
   das dadurch gekennzeichnet ist, daß man im Reaktionsschritt b) die Isomerisierung des Gemisches aus 13-(Z)- und 11,13-di-(Z)-Retinsäure in einem aliphatischen oder cycloaliphatischen Kohlenwasserstoff, einem halogenierten aliphatischen oder aromatischen Kohlenwasserstoff oder einem C₁- bis C₄-Alkanol als Lösungsmittel, vorzugsweise einem C₁-C₄-Alkanol, in Gegenwart eines Photosensibilisators, vorzugsweise in Gegenwart von Erytrosin B, mit Licht im Wellenlängenbereich zwischen 200 und 600 nm bestrahlt.

Das als Ausgangsverbindung verwendete 5-Hydroxy-4-methyl-2-(5H)-furanon ist eine handelsübliche Verbindung, die durch Acetalspaltung und intramolekulare Cyclisierung aus β-Formyl-crotonsäureesteracetal herstellt werden kann.

Die C₁₅-Triarylphosphoniumsalze, insbesondere die C₁₅-Triphenylphosphoniumsalze, der allgemeinen Formel III sind als essentielle Zwischenprodukte einer der technischen Vitamin-A-Synthesen technisch gut verfügbar.

Zur Durchführung der Wittig-Reaktion in Stufe a) wird mit Vorteil das Lithiumhydroxid im Dimethylformamid (DMF) suspendiert und die Suspension auf 0°C abgekühlt. Zu der abgekühlten Suspension wird zunächst langsam eine Lösung des C₁₅-Triarylphosphoniumsalzes in DMF und danach langsam eine Lösung des 5-Hydroxy-3-methyl-2-(5H)-furanons in DMF zugefügt. Nach 2 bis 10, vorzugsweise 3 bis 5 stündigem Rühren bei Temperaturen von +10 bis -9°C, vorzugsweise +5 bis -5°C, insbesondere +2 bis -2°C, wird das Reaktionswasser mit Eiswasser versetzt. Die Wasser/DMF-Phase wird dann beispielsweise mit Hexan extrahiert, danach angesäuert und mit einem Hexan/Essigsäureethylestergemisch extrahiert. Die organische Phase wird mit Wasser gewaschen und bei ca. 40°C von Lösungsmittel befreit.

Das Lithiumhyroxid verwendet man hierbei in Mengen von 2 bis 6 Mol, vorzugsweise 3 bis 5 Mol pro Mol des C₁₅-Triarylphosphoniumsalzes.

Das DMF verwendet man im allgemeinen insgesamt in Mengen von 2 bis 8 Litern (1), vorzugsweise 3 bis 6 l, bezogen auf 1 Mol C₁₅-Triarylphosphoniumsalz.

Als Extraktionsmittel verwendet man mit Vorteil Hexan bzw. ein Gemisch aus Hexan und Essigsäureethylester. Es können aber auch alle anderen mit Wasser nicht mischbaren organischen Lösungsmittel, wie Ether, aliphatische Kohlenwasserstoffe, halogenierte und aromatische Kohlenwasserstoffe zur Extraktion des Retinsäure-Isomerengemisches verwendet werden.

Das DMF bleibt bei dieser Extraktion in der wäßrigen Phase und hält auch das bei der Wittig-Reaktion gebildete Triphenylphosphinoxid gelöst in der wäßrigen Phase zurück. Hieraus ergibt sich ein weiterer Vorteil gegenüber dem Verfahren gemäß EP 111 325, bei dem das Triphenylphosphinoxid z.T. in die das Wertprodukt enthaltende organische Phase gelangt und hieraus nur mit größerem Aufwand abtrennbar ist.

Bei der oben beschriebenen Wittig-Reaktion fällt ein Gemisch an 13-(Z)- und 11,13-di-(Z)-Retinsäure in Ausbeuten von 95 bis 98 % der Theorie an.

Dieses Gemisch kann dann entweder durch übliche Methoden, beispielsweise durch fraktionierte Kristallisation aufgetrennt und das dabei abgetrennte 11,13-di-(Z)-Isomere in das 13-(Z)-Isomere umgewandelt werden. Mit Vorteil unterwirft man jedoch direkt das erhaltene Isomerengemisch der Isomerisierung.

Als Lösungsmittel für die Isomerisierung kommen die für Photoisomerisierungen allgemein bekannten Lösungsmittel in Betracht. Genannt seien aliphatische und cycloaliphatische Kohlenwasserstoffe sowie halogenierte aliphatische und aromatische Kohlenwasserstoffe. Mit besonderem Vorteil arbeitet man in C₁- bis C₄-Alkanolen, insbesondere in Isopropanol.

Als Photosensibilisatoren kommen leicht verfügbare Verbindungen, die sowohl farbig und/oder fluoreszierend sein können, in Betracht. Genannt seien beispielsweise 2,4,6-Triphenylpyryliumperchlorat; 2,4,6-Triphenylpyryliumpicrat; 2,4,6-Triphenylpyryliumchloroferrat; 2,4,6-Tri-(p-dimethylaminophenyl)pyryliumperchlorat; 2,6-Diphenyl-4-(p-dimethylamino)-pyryliumchlorid; Perylen; Chinizarin; β-Chinophthalen; Fluorescein; Eosin; Rose bengale; Erythrosin; Euchrysinorange; Rhodamin B und andere Rhodamine; Diethylsafranin; Astraphloxin; Pseudoisocyanin und andere Cyanine; Chinolinrot bzw. Basacrylbrilliantrot; Malachitgrün; Methylenblau; Kristallviolett; 1,8-Dihydroxy-4,5-diaminobromanthrachinon; β-Carotin; Tetraphenylporphin und seine Metallkomplexe mit Mg, Zn, Sn, Ni, Cu oder Co; Tetra-(p-methoxyphenyl)-porphin und seine Metallkomplexe; Chlorophyll; Hämatoporphyrin und Mischungen von zwei oder mehreren der genannten Sensibilisatoren.

Mit besonderem Vorteil arbeitet man mit Erytrosin B.

Als Lichtquelle für Licht im Wellenlängenbereich zwischen 200 und 600 nm kommen Hg-Dampf-Lampen in verschiedenen Druckbereichen (Hoch-, Mittel- und Niederdruck-Lampen) in Betracht.

Die Isomerisierung kann im Temperaturbereich von -10 bis 60°C, vorzugsweise 0 bis 30°C durchgeführt werden. Mit besonderem Vorteil arbeitet man bei Raumtemperatur d.h. ca. 20°C.

Die Reaktionszeit für die Isomerisierung beträgt im allgemeinen 10 bis 60 Minuten, vorzugsweise 15 bis 35 Minuten.

Die so erhaltene 13-(Z)-Retinsäure kristallisiert aus der Isomerisierungslösung aus und kann in mindestens 45 %iger und bis zu 99 %iger Reinheit daraus isoliert werden. Die verbleibende Mutterlauge kann erneut in die Isomerisierungsvorrichtung zurückgeführt werden.

### Beispiel 1

a) 32,2 g LiOH wurden in 350 ml Dimethylformamid (DMF) suspendiert. Man kühlte den Ansatz auf 0°C ab und tropfte innerhalb von 30 Minuten (min) eine Lösung aus 143 g 3-Methyl-5-(2,6,6-trimethyl-1-cyclohexan-1-yl)-2,4-pentadienyl)-triphenylphosphoniumhydrogensulfat in 333 g DMF zu. Anschließend tropfte man innerhalb von 30 min 52,3 g 5-Hydroxy-4-methyl-2-(5H)-furanon (Butenolid) in 263 g DMF zu und ließ den Ansatz für 4 Stunden (h) bei 0°C rühren. Danach wurden 1050 ml Eiswasser zugesetzt. Die Wasser/DMF-Phase wurde 3 ml mit 560 ml n-Hexan extrahiert, bevor sie mit 42 ml konz. Schwefelsäure auf einen pH 3 eingestellt wurde. Nachfolgend wurde mit 2 ml 800 ml Hexan/Essigsäureethylester (4:1) extrahiert. Die organische Phase wurde 2 mal mit 350 ml Wasser gewaschen und unter vermindertem Druck bei 40°C vollständig vom Lösungsmittel befreit. Der Rückstand wurde 5 h unter vermindertem Druck bei 75°C getrocknet und lieferte 76,7 g Retinsäure in Form eines Isomerengemisches bestehend aus 13-(Z)- und 13,11-di-(Z)-Retinsäure. Das entspricht einer Ausbeute von 97,5 % der Theorie.
b) 62,5 g des gemäß a) erhaltenen Retinsäure-Isomerengemisches wurde in 750 ml Isopropanol gelöst und mit 1 ml einer 1 %igen Lösung von Erytrosin B in Methanol versetzt. Das erhaltene Gemisch pumpte man bei Raumtemperatur (RT) unter Bestrahlung mit UV-Licht (Wellenlänge 200 bis 600 nm) in einer Umlaufapparatur um. Nach 30 min war die Isomerisierung beendet. Der erhaltene Kristallbrei wurde abfiltriert und mit 120 ml gekühltem n-Heptan gewaschen. Nach Trocknung in Stickstoffstrom wurde das Wertprodukt in 99 % Reinheit erhalten. Die resultierende Mutterlauge kann der Photoisomerisierung erneut unter Ausbildung von Wertprodukten zugeführt werden. Ausbeute 50 % an 13-(Z)-Retinsäure (kristallin) bezogen auf ein gesetztes C₁₅-Triphenylphosphoniumsalz.

### Beispiel 2

57,3 g eines analog Beispiel la hergestellten Gemisches aus 13-(Z)- und 13,11-di-(Z)-Retinsäure wurden in 750 ml Methanol gelöst und die Lösung mit 1,2 ml einer 1 %igen Erythrosin-B-Lösung in Methanol versetzt. Das so erhaltene Reaktionsgemisch wurde bei RT in einem Photoreaktor im Kreis gepumpt und mit Licht einer Wellenlänge von 200 bis 600 nm bestrahlt. Nach 30 min war die Isomerisierung beendet. Die gewünschte 13-(Z)-Retinsäure kristallisierte aus der Reaktionslösung aus und wurde abfiltriert. Man erhielt so 27,5 g kristallines Wertprodukt entsprechend einer Ausbeute von 48 %.

### Beispiel 3 (Vergleichsbeispiel)

20,4 g 3-Methyl-5-(2,6,6-trimethyl-1-cyclohexan-1-yl)-2,4-pentadienyl)-triphenylphosphoniumhydrogensulfat wurden in 50 ml DMF gelöst und innerhalb von 30 min bei 0°C zu einer Suspension von 12,5 g KOH in 50 ml DMF zugetropft. Anschließend wurden bei 0°C 7,4 g Butenolid in 50 ml DMF zugetropft und das Reaktionsgemisch nach 30 min bei 0°C gerührt und anschließend mit 300 ml Wasser versetzt und durch Ansäuern mit konz. Schwefelsäure auf einen pH-Wert von 2 gebracht.

Anschließend wurde analog Beispiel 1 mit einem Hexan-Essigsäureethylestergemisch (4:1) extrahiert. Man erhielt 0,87 g Retinsäure in Form eines Isomerengemisches bestehend aus 13-(Z)- und 13,11-di-(Z)-Retinsäure.

## Patentansprüche

1. Verfahren zur Herstellung von 13-(Z)-Retinsäure der allgemeinen Formel I 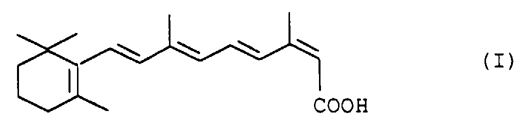 durch
a) Umsetzen von 5-Hydroxy-4-methyl-2-(5H)-furanon der Formel II 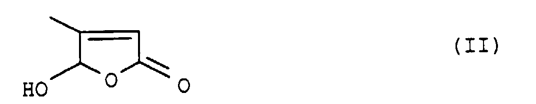 mit einem C₁₅-Triarylphosphoniumsalz der allgemeinen Formel III 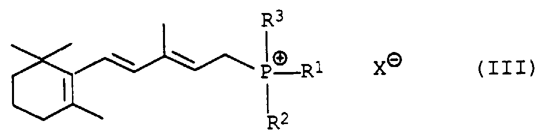 in der R¹, R² und R³ für einen Arylrest stehen und X^{⊖} für Halogen oder (HSO₄)⁻ steht, in Gegenwart eines Alkalimetallhydroxids in einem organischen Lösungsmittel und
b) anschließende partielle Isomerisierung des so erhaltenen Gemisches aus 13-(Z)- und 11,13-di-(Z)-Retinsäure,
dadurch gekennzeichnet, daß man im Reaktionsschritt a) das 5-Hydroxy-4-methyl-2-(5H)-furanon )-furanon der Formel II mit einem Gemisch aus dem C₁₅-Triarylphosphoniumsalz der Formel III und 2 bis 6 Mol Lithiumhydroxid pro Mol des C₁₅-Triarylphosphoniumsalzes der Formel III als Alkalimetallhydroxid in 2 bis 6 Litern Dimethylformamid pro Mol des C₁₅-Triarylphosphoniumsalzes der Formel III als Lösungsmittel bei Temperaturen von 10 bis -9°C umsetzt.

2. Verfahren zur Herstellung von 13-(Z)-Retinsäure gemäß Anspruch 1, dadurch gekennzeichnet, daß man im Verfahrensschritt a) das 5-Hydroxy-4-methyl-2-(5H)-furanon mit dem C₁₅-Triarylphosphoniumsalz bei Temperaturen von +5 bis -5°C umsetzt.

3. Verfahren zur Herstellung von 13-(Z)-Retinsäure gemäß Anspruch 1, dadurch gekennzeichnet, daß man im Verfahrensschritt a) das 5-Hydroxy-4-methyl-2-(5H)-furanon mit einem C₁₅-Triphenylphosphoniumhydrogensulfat als C₁₅-Triarylphosphoniumsalz umsetzt.

4. Verfahren zur Herstellung von 13-(Z)-Retinsäure gemäß Anspruch 1, dadurch gekennzeichnet, daß man im Verfahrensschritt b) zur Isomerisierung des Gemisches aus 13-(Z)- und 11,13-di-(Z)-Retinsäure dieses Gemisch in einem aliphatischen oder cycloaliphatischen Kohlenwasserstoff, einem halogenierten aliphatischen oder aromatischen Kohlenwasserstoff oder einem C₁- bis C₄-Alkanol als Lösungsmittel in Gegenwart eines Photosensibilisators mit Licht im Wellenlängenbereich zwischen 200 und 600 nm bestrahlt.

5. Verfahren gemäß Anspruch 4 oder 7, dadurch gekennzeichnet, daß man für die Isomerisierung des Gemisches aus 13-(Z)- und 11,13-di-(Z)-Retinsäure ein C₁- bis C₄-Alkanol als Lösungsmittel verwendet.

6. Verfahren gemäß Anspruch 4 oder 7, dadurch gekennzeichnet, daß man für die Isomerisierung des Gemisches aus 13-(Z)- und 11,13-di-(Z)-Retinsäure Erytrosin-B als Photosensibilisator verwendet.

7. Verfahren zur Herstellung von 13-(Z)-Retinsäure der allgemeinen Formel I gemäß Oberbegriff von Anspruch 1, dadurch gekennzeichnet, daß man im Reaktionsschritt b) zur Isomerisierung des Gemisches aus 13-(Z)- und 11,13-di-(Z)-Retinsäure dieses Gemisch in einem aliphatischen oder cycloaliphatischen Kohlenwasserstoff, einem halogenierten aliphatischen oder aromatischen Kohlenwasserstoff oder einem C₁-bis C₄-Alkanol als Lösungsmittel in Gegenwart eines Photosensiblisators mit Licht im Wellenlängenbereich zwischen 200 und 600 nm bestrahlt.

8. Verfahren gemäß Anspruch 7, dadurch gekennzeichnet, daß man im Reaktionsschritt b) zur Isomerisierung des Gemisches aus 13-(Z)- und 11,13-di-(Z)-Retinsäure dieses Gemisch in einem C₁- bis C₄-Alkanol als Lösungsmittel in Gegenwart eines Photosensibilisation mit Licht im Wellenlängenbereich zwischen 200 und 600 nm bestrahlt.

## Claims

1. A process for preparing 13-(Z)-retinoic acid of the formula I 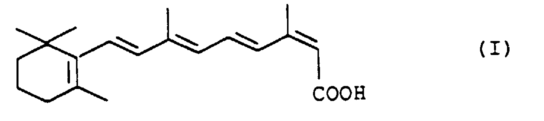 by
a) reacting 5-hydroxy-4-methyl-2(5H)-furanone of the formula II 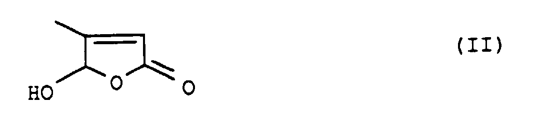 with a C₁₅-triarylphosphonium salt of the formula III 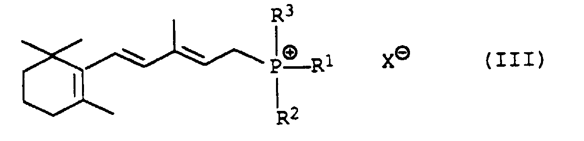 where R¹, R² and R³ are each aryl and X^{⊖} is halogen or HSO₄⁻, in the presence of an alkali metal hydroxide in an organic solvent and
b) subsequently partially isomerizing the resulting mixture of 13-(Z)- and 11,13-di-(Z)-retinoic acid,
wherein the 5-hydroxy-4-methyl-2(5H)-furanone of the formula II is reacted in step a) with a mixture of the C₁₅-triarylphosphonium salt of the formula III and from 2 to 6 mol of lithium hydroxide per mol of the C₁₅-triarylphosphonium salt of the formula III as alkali metal hydroxide in from 2 to 6 liters of dimethylformamide per mol of the C₁₅-triarylphosphonium salt of the formula III as solvent at from 10 to -9°C.

2. A process for preparing 13-(Z)-retinoic acid as claimed in claim 1, wherein the 5-hydroxy-4-methyl-2 (5H)-furanone is reacted in step a) with the C₁₅-triarylphosphonium salt at from +5 to -5°C.

3. A process for preparing 13-(Z)-retinoic acid as claimed in claim 1, wherein the 5-hydroxy-4-methyl-2 (5H)-furanone is reacted in step a) with a C₁₅-triphenylphosphonium hydrogen sulfate as C₁₅-triarylphosphonium salt.

4. A process for preparing 13-(Z)-retinoic acid as claimed in claim 1, wherein the isomerization of the mixture of 13-(Z)- and 11,13-di-(Z)-retinoic acid in step b) is carried out by irradiating this mixture in an aliphatic or cycloaliphatic hydrocarbon, a halogenated aliphatic or aromatic hydrocarbon or a C₁- to C₄-alkanol as solvent in the presence of a photosensitizer with light in the wavelength range from 200 to 600 nm.

5. A process as claimed in claim 4 or 7, wherein a C₁-C₄-alkanol is used as solvent for the isomerization of the mixture of 13-(Z)- and 11,13-di-(Z)-retinoic acid.

6. A process as claimed in claim 4 or 7, wherein erythrosin B is used as photosensitizer for the isomerization of the mixture of 13-(Z)- and 11,13-di-(Z)-retinoic acid.

7. A process for preparing 13-(Z)-retinoic acid of the formula I as set forth in the preamble of claim 1, wherein the isomerization of the mixture of 13-(Z)- and 11,13-di-(Z)-retinoic acid in step b) is carried out by irradiating this mixture in an aliphatic or cycloaliphatic hydrocarbon, a halogenated aliphatic or aromatic hydrocarbon or a C₁- to C₄-alkanol as solvent in the presence of a photosensitizer with light in the wavelength range from 200 to 600 nm.

8. A process as claimed in claim 7, wherein the isomerization of the mixture of 13-(Z)- and 11,13-di-(Z)-retinoic acid in step b) is carried out by irradiating this mixture in a C₁- to C₄-alkanol as solvent in the presence of a photoinitiator with light in the wavelength range from 200 to 600 nm.

## Revendications

1. Procédé de préparation d'acide 13-(Z)-rétinoïque de formule générale I 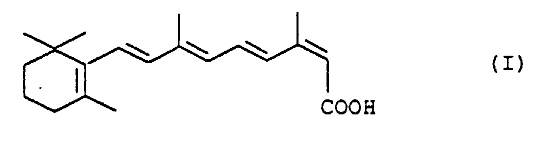 par
a) réaction de 5-hydroxy-4-méthyl-2-(5H)-furanone de formule II 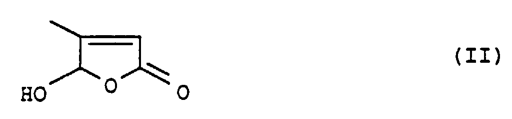 avec un sel de triaryl (en C15)-phosphonium de formule générale III 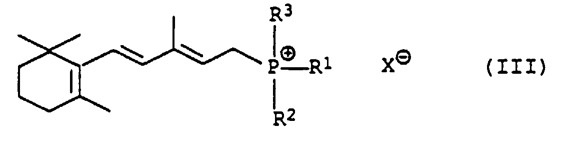 dans laquelle R¹, R² et R³ sont mis pour un reste aryle et X^{⊖} pour halogène ou (HSO₄)⁻, en présence d'un hydroxyle de métal alcalin, dans un solvant organique et
b) ensuite isomérisation partielle du mélange ainsi obtenu d'acide 13-(Z)- et 11,13-di-(Z)-rétinoïque
caractérisé par le fait que, dans l'étape de réaction a), on fait réagir la 5-hydroxy-4-méthyl-2-(5H)-furanone de formule II avec un mélange du sel de triaryl (en C15)-phosphonium de formule III et 2 à 6 moles d'hydroxyde de lithium par mole du sel de triaryl (en C15)-phosphonium de formule III, comme hydroxyde de métal alcalin, dans 2 à 6 litres de diméthylformamide par mole du sel de triaryl (en C15)-phosphonium de formule III comme solvant à des températures de 10 à -9°C.

2. Procédé de préparation d'acide 13-(Z)-rétinoïque selon la revendication 1, caractérisé par le fait que, dans l'étape a) du procédé, on fait réagir la 5-hydroxy-4-méthyl-2-(5H)-furanone avec le sel de triaryl (en C15)-phosphonium à des températures de +5 à -5°C.

3. Procédé de préparation d'acide 13-(Z)-rétinoïque selon la revendication 1, caractérisé par le fait que, dans l'étape a) du procédé, on fait réagir la 5-hydroxy-4-méthyl-2-(5H)-furanone avec un hydrogénosulfate de triphényl (en C15)phosphonium comme sel de triaryl (en C15)-phosphonium.

4. Procédé de préparation d'acide 13-(Z)-rétinoïque selon la revendication 1, caractérisé par le fait que, dans l'étape b) du procédé pour isomériser le mélange d'acide 13-(Z)- et 11,13-di-(Z)-rétinoïque, on irradie ce mélange dans un hydrocarbure aliphatique ou cycloaliphatique, un hydrocarbure aliphatique ou aromatique halogéné ou un alcanol en C1-C4, comme solvant, en présence d'un photosensibilisateur, avec une lumière du domaine des longueurs d'onde comprises entre 200 et 600 nm.

5. Procédé selon la revendication 4 ou 7, caractérisé par le fait que, pour l'isomérisation du mélange d'acide 13-(Z)- et 11,13-di-(Z)-rétinoïque, on utilise un alcanol en C1-C4 comme solvant.

6. Procédé selon la revendication 4 ou 7, caractérisé par le fait que, pour l'isomérisation du mélange d'acide 13-(Z)- et 11,13-di-(Z)-rétinoïque, on utilise l'érytrosine B comme photosensibilisateur.

7. Procédé de préparation d'acide 13-(Z)-rétinoïque de formule générale I, selon le préambule de la revendication 1,
caractérisé par le fait que, dans l'étape (b) du procédé, pour isomériser le mélange d'acide 13-(Z)- et 11,13-di-(Z)-rétinoïque, on irradie ce mélange dans un hydrocarbure aliphatique ou cycloaliphatique, un hydrocarbure aliphatique ou aromatique halogéné ou un alcanol en C1-C4, comme solvant, en présence d'un photosensibilisateur, avec une lumière du domaine des longueurs d'onde comprises entre 200 et 600 nm.

8. Procédé selon la revendication 7, caractérisé par le fait que, dans l'étape (b) du procédé, pour l'isomérisation du mélange, on utilise un alcanol en C1-C4 comme solvant et en présence d'un photosensibilisateur, avec une lumière du domaine des longueurs d'onde comprises entre 200 et 600 nm.
